(19) Europäisches Patentamt
European Patent Office
Office européen des brevets

(11) Veröffentlichungsnummer: **0 008 056**
**B1**

(12) # EUROPÄISCHE PATENTSCHRIFT

(45) Veröffentlichungstag der Patentschrift: **16.09.81**

(51) Int. Cl.³: **C 07 D 231/12,** C 07 D 231/16

(21) Anmeldenummer: **79102705.5**

(22) Anmeldetag: **30.07.79**

(54) **Neue N-substituierte Pyrazolderivate, Verfahren zu ihrer Herstellung und dabei erhaltene neue N-Hydroxyalkylpyrazole.**

(30) Priorität: **10.08.78 DE 2835158**

(43) Veröffentlichungstag der Anmeldung: **20.02.80 Patentblatt 80/4**

(45) Bekanntmachung des Hinweises auf die Patenterteilung: **16.09.81 Patentblatt 81/37**

(84) Benannte Vertragsstaaten: **BE CH DE FR GB IT NL**

(56) Entgegenhaltungen:
**DE-A-1 620 016**
**DE-A-2 107 715**
**DE-B-1 296 637**
**US-A-3 293 261**

(73) Patentinhaber: **BAYER AG, *Zentralbereich Patente,* Marken und Lizenzen, D-5090 Leverkusen 1, Bayerwerk (DE)**

(72) Erfinder: **Schmidt, Thomas, Dr., Am Bandenfeld 72, D-5657 Haan (DE)**
Erfinder: **Thomas, Rudolf, Dr., Wilkhausstrasse 129, D-5600 Wuppertal 2 (DE)**

**EP 0 008 056 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue N-substituierte Pyrazolderivate, Verfahren zu ihrer Herstellung und dabei erhaltene neue N-Hydroxyalkylpyrazole

Die vorliegende Anmeldung bertrifft neue substituierte Pyrazol-Derivate, ein Verfahren zu ihrer Herstellung sowie ihre Verwendung als Zwischenprodukte zur Synthese von substituierten N-Pyrazolylmethyl-halogenacetaniliden, welche herbizide Eigenschaften besitzen.

Es ist bereits bekannt geworden, dass man 2,6-Diethyl-N-methoxymethyl-chloracetanilid zur selektiven Unkrautbekämpfung verwenden kann (vgl. R. Wegler, Chemie der Pflanzenschutz- und Schädlingsbekämpfungsmittel, Band 5, Seite 255, Springer-Verlag [1977]). Diese Verbindung ist jedoch nicht immer ausreichend wirksam und in ihrer Selektivität nicht immer ganz befriedigend.

Weiterhin ist bereits bekannt geworden, dass zahlreiche N-substituierte Halogenacetanilide, in denen das Stickstoffatom des Anilinteiles über eine $CH_2$-Gruppe mit einem gegebenenfalls substituierten Azol über ein N-Atom des Azol-Restes verbunden ist, gute herbizide Eigenschaften besitzen (vergleiche FR-A 2368476). Die selektive herbizide Wirksamkeit dieser Stoffe lässt jedoch in manchen Fällen zu wünschen übrig.

Es wurden nun als neue Verbindungen die substituierten Pyrazol-Derivate der Formel

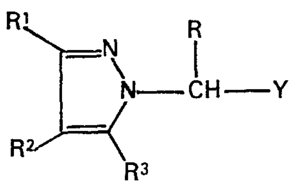
(I)

in welcher R für Alkyl mit 1 bis 8 Kohlenstoffatomen, Cycloalkyl mit 3 bis 6 Kohlenstoffatomen, Halogenalkyl mit bis zu 2 Kohlenstoffatomen und bis zu 3 Halogenatomen, Alkoxyalkyl mit 1 bis 4 Kohlenstoffatomen im Alkylteil und 1 bis 4 Kohlenstoffatomen im Alkoxyteil, für Alkenyl mit 2 bis 4 Kohlenstoffatomen, Alkinyl mit 2 bis 4 Kohlenstoffatomen oder für gegebenenfalls durch Halogen, Alkyl mit 1 bis 4 Kohlenstoffatomen, Halogenalkyl mit bis zu 2 Kohlenstoffatomen und bis zu 3 Halogenatomen, Alkoxy mit bis zu 2 Kohlenstoffatomen, Alkylthio mit bis zu 2 Kohlenstoffatomen, Cyano und/oder Nitro-substituiertes Phenyl steht, $R^1$, $R^2$ und $R^3$ gleich oder verschieden sind und für Wasserstoff, Alkyl mit 1 bis 4 Kohlenstoffatomen, Haloger oder Alkoxy mit 1 bis 4 Kohlenstoffatomen stehen und Y für Fluor, Chlor, Brom, Jod, Alkylsulfonyl mit 1 bis 4 Kohlenstoffatomen im Alkylteil oder für gegebenenfalls durch Alkyl mit 1 bis 4 Kohlenstoffatomen und/oder Halogensubstituiertes Phenylsulfonyl steht, und deren Säureadditionssalze gefunden.

Weiterhin wurde gefunden, dass man substituierte Pyrazol-Derivate der Formel (I) erhält, wenn man Pyrazole der Formel

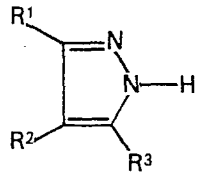
(II)

in welcher $R^1$, $R^2$ und $R^3$ die oben angegebene Bedeutung haben, mit Aldehyden der Formel

$$O = CH\text{-}R \qquad (III)$$

in welcher R die oben angegebene Bedeutung hat, gegebenenfalls in Gegenwart eines Lösungsmittels umsetzt und die dabei entstehenden Hydroxy-Pyrazol-Derivate der Formel

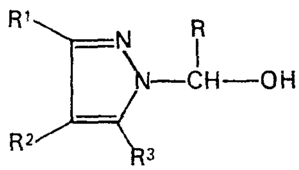
(IV)

in welcher R, $R^1$, $R^2$ und $R^3$ die oben angegebene Bedeutung haben, direkt oder gegebenenfalls nach Isolierung mit einem Halogenierungsmittel bzw. einem Sulfonylierungsmittel, gegebenenfalls in Gegenwart eines Lösungsmittels umsetzt. Die Verbindungen der Formel (I) fallen hierbei in der Regel als Säureadditions-Salze an.

Ausserdem wurden als neue Verbindungen die Hydroxy-Pyrazol-Derivate der Formel

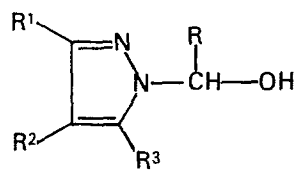
(IVa)

in welcher R für Alkyl mit 1 bis 8 Kohlenstoffatomen oder für Halogenalkyl mit bis zu 2 Kohlenstoffatomen und bis zu 3 Halogenatomen steht, $R^1$ für Wasserstoff oder Alkyl mit 1 bis 4 Kohlenstoffatomen steht, $R^2$ für Wasserstoff oder Halogen steht und $R^3$ für Wasserstoff oder Alkyl mit 1 bis 4 Kohlenstoffatomen steht, gefunden.

Die erfindungsgemässen substituierten Pyrazol-Derivate der Formel (I) eignen sich als Zwischenprodukte zur Synthese von substituierten N-Pyrazolylmethyl-halogenacetaniliden, welche herbizide Eigenschaften besitzen.

Überraschenderweise sind die substituierten N-Pyrazolyl-methyl-halogenacetanilide, die sich aus den erfindungsgemässen Pyrazol-Derivaten der Formel (I) durch Umsetzung mit Halogenacetaniliden herstellen lassen, dem bekannten 2,6-Diethyl-N-methoxymethyl-chloracetanilid bei gleichwertiger Unkrautwirkung durch bessere Einsatzmöglichkeiten als selektive Unkrautbekämpfungsmittel überlegen.

Ferner übertreffen die aus den erfindungsgemässen Stoffen der Formel (I) herstellbaren substituierten N-Pyrazolyl-methyl-halogenacetanilide auch konstitutionell ähnliche Verbindungen, die in der FR-A 2 368 476 offenbart werden, bezüglich ihrer selektiven herbiziden Wirksamkeit. So lässt sich das 2,6-Dimethyl-N-(pyrazol-1-yl-eth-1-yl)-chloracetanilid besser zur selektiven Unkrautbekämpfung in Sinapis einsetzen als das 2,6-Diethyl-N-(pyrazol-1-yl-methyl)-chloracetanilid, das in der FR-A 2 368 476 beschrieben wird. Darüberhinaus besitzen das 2-Ethyl-6-methyl-N-(pyrazol-1-yl-eth-1-yl)-chloracetanilid und das 2,6-Dimethyl-N-(pyrazol-1-yl-eth-1-yl)-chloracetanilid überraschenderweise bei der Unkrautbekämpfung in Weizen, Baumwolle, Mais und Rüben bessere selektive herbizide Eigenschaften als das vorbekannte 2,6-Diethyl-N-(1,2,4-triazol-1-yl-methyl)-chloracetanilid. Die erfindungsgemässen Stoffe stellen somit als Zwischenprodukte zur Synthese hochwertiger Herbizide eine wesentliche Bereicherung der Technik dar.

Die unsymmetrisch substituierten Pyrazol-Derivate der Formel (I) treten aufgrund tautomerer Strukturen in den zur Herstellung verwendeten Ausgangsstoffen in zwei isomeren Formen auf, die sich formelmässig wie folgt veranschaulichen lassen:

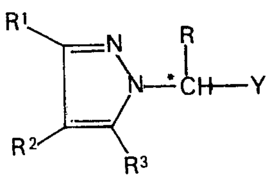

und

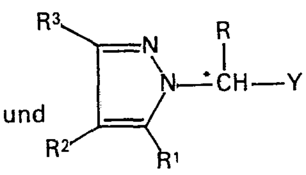

(a) (b)

Das Isomeren-Verhältnis wird im wesentlichen von der Art der Pyrazol-Substituenten bestimmt. Ausserdem können die Verbindungen der Formel (I) als optische Isomere vorliegen (vgl. durch * markiertes Kohlenstoffatom). Meist fallen jedoch Gemische an, die alle Isomeren enthalten. Die Formel (I) umfasst sowohl die Stellungsisomeren als auch die optischen Isomeren.

Die obigen Ausführungen über Strukturisomere und optische Isomere der Pyrazol-Derivate der Formel (I) gelten analog für die Verbindungen der Formel (IV) und für die substituierten N-Pyrazolyl-methyl-halogenacetanilide der Formel (V).

Die Formeln (IV), (IVa) und (V) umfassen ebenfalls sowohl die Stellungsisomeren als auch die optischen Isomeren.

Die Verbindungen der Formel (IV) stehen im chemischen Gleichgewicht mit ihren Ausgangsstoffen und sind deshalb nur in Einzelfällen isolierbar.

Ganz besonders bevorzugt sind diejenigen Verbindungen der Formel (I), in denen R für Methyl, Ethyl, n-Propyl, Isopropyl, Isobutyl, sek.-Butyl, n-Butyl, Trifluormethyl, Chlormethyl, Dichlormethyl, Trichlormethyl, Tribrommethyl, Vinyl, Allyl, Propen-1-yl, Ethinyl, Propargyl, Phenyl, Chlorphenyl, Dichlorphenyl, Methylphenyl, Dimethylphenyl oder Chlormethylphenyl oder Nitrophenyl steht; $R^1$, $R^2$ und $R^3$ gleich oder verschieden sind und für Wasserstoff, Methyl, Chlor, Brom oder Methoxy stehen; und Y für Chlor, Brom, Methylsulfonyl, Phenylsulfonyl, p-Methylphenylsulfonyl oder p-Bromphenylsulfonyl steht.

Als Beispiele seien genannt:

1-(1'-Brom(chlor)ethyl)-pyrazol
1-(1'-Brom(chlor)ethyl)-4-chlor-pyrazol
1-(1'-Brom(chlor)ethyl)-2-methyl-pyrazol
1-(1'-Brom(chlor)ethyl)-5-methyl-pyrazol
1-(1'-Brom(chlor)ethyl)-3,5-dimethyl-pyrazol
1-(1'-Brom(chlor)ethyl)-4-chlor-3,5-dimethyl-pyrazol
1-(1'-Brom(chlor)ethyl)-4-methoxy-pyrazol
1-(1'-Brom(chlor)propyl)-pyrazol
1-(1'-Brom(chlor)propyl)-4-chlor-pyrazol
1-(1'-Brom(chlor)propyl)-3-methyl-pyrazol
1-(1'-Brom(chlor)propyl)-5-methyl-pyrazol
1-(1'-Brom(chlor)propyl)-3,5-dimethyl-pyrazol
1-(1'-Brom(chlor)propyl)-4-chlor-3,5-dimethyl-pyrazol
1-(1'-Brom(chlor)propyl)-4-methoxy-pyrazol
1-(1'-Brom(chlor)butyl)-pyrazol
1-(1'-Brom(chlor)butyl)-4-chlorpyrazol
1-(1'-Brom(chlor)butyl)-2-methyl-pyrazol
1-(1'-Brom(chlor)butyl)-5-methyl-pyrazol
1-(1'-Brom(chlor)butyl)-3,5-dimethyl-pyrazol
1-(1'-Brom(chlor)butyl)-4-chlor-3,5-dimethyl-pyrazol
1-(1'-Brom(chlor)butyl)-4-methoxy-pyrazol
1-(1'-Brom(chlor)-2'-methyl-propyl)-pyrazol
1-(1'-Brom(chlor)-2'-methyl-propyl)-4-chlor-pyrazol
1-(1'-Brom(chlor)-2'-methyl-propyl)-3-methyl-pyrazol
1-(1'-Brom(chlor)-2'-methyl-propyl)-5-methyl-pyrazol
1-(1'-Brom(chlor)-2'-methyl-propyl)-3,5-dimethyl-pyrazol
1-(1'-Brom(chlor)-2'-methyl-propyl)-4-chlor-3,5-dimethyl-pyrazol
1-(1'-Brom(chlor)-2'-methyl-propyl)-4-methoxy-pyrazol
1-(1'-Brom(chlor)pentyl)-pyrazol
1-(1'-Brom(chlor)pentyl)-4-chlor-pyrazol
1-(1'-Brom(chlor)pentyl)-3-methyl-pyrazol
1-(1'-Brom(chlor)pentyl)-5-methylpyrazol
1-(1'-Brom(chlor)pentyl)-3,5-dimethyl-pyrazol
1-(1'-Brom(chlor)pentyl)-4-chlor-3,5-dimethyl-pyrazol
1-(1'-Brom(chlor)pentyl)-4-methoxy-pyrazol
1-(Brom(chlor)-cyclopropyl-methyl)-pyrazol
1-(Brom(chlor)-cyclopropyl-methyl)-4-chlor-pyrazol
1-(Brom(chlor)-cyclopropyl-methyl)-3-methyl-pyrazol
1-(Brom(chlor)-cyclopropyl-methyl)-5-methyl-pyrazol
1-(Brom(chlor)-cyclopropyl-methyl)-3,5-dimethyl-pyrazol
1-(Brom(chlor)-cyclopropyl-methyl)-4-chlor-3,5-dimethyl-pyrazol

1-(Brom(chlor)-cyclopropyl-methyl)-4-methoxy-pyrazol
1-(Brom(chlor)-(2',4'-dichlorphenyl)-methyl)-pyrazol
1-(Brom(chlor)-(2',4'-dichlorphenyl)-methyl)-4-chlor-pyrazol
1-(Brom(chlor)-(2',4'-dichlorphenyl)-methyl)-3-methyl-pyrazol
1-(Brom(chlor)-(2',4'-dichlorphenyl)-methyl)-5-methyl-pyrazol
1-(Brom(chlor)-(2',4'-dichlorphenyl)-methyl)-3,5-dimethyl-pyrazol
1-(Brom(chlor)-(2',4'-dichlorphenyl)-methyl)-4-chlor-3,5-dimethyl-pyrazol
1-(Brom(chlor)-(2',4'-dichlorphenyl)-methyl)-4-methoxy-pyrazol
1-(1',2',2',2'-Tetrabrom(chlor)-ethyl)-pyrazol
1-(1',2',2',2'-Tetrabrom(chlor)-ethyl)-4-chlor-pyrazol
1-(1',2',2',2'-Tetrabrom(chlor)-ethyl)-3-methyl-pyrazol
1-(1',2',2',2'-Tetrabrom(chlor)-ethyl)-5-methyl-pyrazol
1-(1',2',2',2'-Tetrabrom(chlor)-ethyl)-3,5-dimethyl-pyrazol
1-(1',2',2',2'-Tetrabrom(chlor)-ethyl)-4-chlor-3,5-dimethyl-pyrazol
1-(1',2',2',2'-Tetrabrom(chlor)-ethyl)-4-methoxy-pyrazol

Als Beispiele für Hydroxy-pyrazol-Derivate der Formel (IV) seien im einzelnen genannt:

1-(1'-Hydroxy-ethyl)-pyrazol
1-(1'-Hydroxy-ethyl)-4-chlor-pyrazol
1-(1'-Hydroxy-ethyl)-5-methyl-pyrazol
1-(1'-Hydroxy-ethyl)-3,5-dimethyl-pyrazol
1-(1'-Hydroxy-ethyl)-4-chlor-3,5-dimethyl-pyrazol
1-(1'-Hydroxy-propxl)-pyrazol
1-(1'-Hydroxy-propyl)-4-chlor-pyrazol
1-(1'-Hydroxy-propyl)-3-methyl-pyrazol
1-(1'-Hydroxy-propyl)-5-mezthyl-pyrazol
1-(1'-Hydroxy-propyl)-3,5-dimethyl-pyrazol
1-(1'-Hydroxy-propyl)-4-chlor-3,5-dimethyl-pyrazol
1-(1'-Hydroxy-butyl)-pyrazol
1-(1'-Hydroxy-butyl)-4-chlor-pyrazol
1-(1'-Hydroxy-butyl)-5-methyl-pyrazol
1-(1'-Hydroxy-butyl)-3,5-dimethyl-pyrazol
1-(1'-Hydroxy-butyl)-4-chlor-3,5-dimethyl-pyrazol
1-(1'-Hydroxy-2'-methyl-propyl)-pyrazol
1-(1'-Hydroxy-2'-methyl-propyl)-4-chlor-pyrazol
1-(1'-Hydroxy-2'-methyl-propyl)-3-methyl-pyrazol
1-(1'-Hydroxy-2'-methyl-propyl)-5-methyl-pyrazol
1-(1'-Hydroxy-2'-methyl-propyl)-3,5-dimethyl-pyrazol
1-(1'-Hydroxy-2'-methyl-propyl)-4-chlor-3,5-dimethyl-pyrazol
1-(1'-Hydroxy-pentyl)-pyrazol
1-(1'-Hydroxy-pentyl)-4-chlor-pyrazol
1-(1'-Hydroxy-pentyl)-3-methyl-pyrazol
1-(1'-Hydroxy-pentyl)-5-methyl-pyrazol
1-(1'-Hydroxy-pentyl)-3,5-dimethyl-pyrazol
1-(1'-Hydroxy-pentyl)-4-chlor-3,5-dimethyl-pyrazol
1-(1'-Hydroxy-2',2',2'-tribrom(chlor)-ethyl)-pyrazol
1-(1'-Hydroxy-2',2',2'-tribrom(chlor)-ethyl)-4-chlor-pyrazol
1-(1'-Hydroxy-2',2',2'-tribrom(chlor)-ethyl)-3-methyl-pyrazol
1-(1'-Hydroxy-2',2',2'-tribrom(chlor)-ethyl)-5-methyl-pyrazol
1-(1'-Hydroxy-2',2',2'-tribrom(chlor)-ethyl)-3,5-dimethyl-pyrazol
1-(1'-Hydroxy-2',2',2'-tribrom(chlor)-ethyl)-4-chlor-3,5-dimethyl-pyrazol

Verwendet man beispielsweise ?yrazol und Acetaldehyd als Ausgangsstoffe und Thionylchlorid als Halogenierungsmittel, so kann der Reaktionsablauf bei dem erfindungsgemässen Verfahren durch das folgende Formelschema wiedergegeben werden:

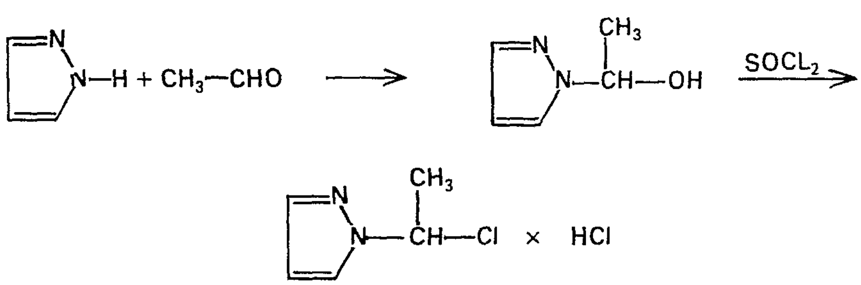


Die bei der Durchführung des erfindungsgemässen Verfahrens als Ausgangsstoffe benötigten Pyrazole sind durch die Formel (II) allgemein definiert. In der Formel (II) stehen $R^1$, $R^2$ und $R^3$ für diejenigen Reste, die bereits im Zusammenhang mit der Beschreibung der erfindungsgemässen Stoffe der Formel (I) für $R^1$, $R^2$ und $R^3$ genannt wurden.

Die Verbindungen der Formel (II) sind allgemein bekannte Verbindungen der organischen Chemie. Als Beispiele seien im einzelnen genannt:

Pyrazol; 4-Chlorpyrazol; 3-Methylpyrazol; 5-Methylpyrazol; 3,5-Dimethylpyrazol; 4-Chlor-3,5-dimethylpyrazol und 4-Methoxypyrazol.

Die bei der Durchführung des erfindungsgemässen Verfahrens weiterhin als Ausgangsstoffe benötigten Aldehyde sind durch die Formel (III) allgemein definiert. In dieser Formel steht R für diejenigen Reste, die bereits im Zusammenhang

mit der Beschreibung der erfindungsgemässen Stoffe der Formel (I) für R genannt wurden.

Die Verbindungen der Formel (III) sind allgemein bekannte Verbindungen der organischen Chemie. Als Beispiele seien im einzelnen genannt:

Acetaldehyd; Propionaldehyd; n-Butyraldehyd; i-Butyraldehyd; Methoxyacetaldehyd; Chloral; Benzaldehyd; 3,4-Dichlorbenzaldehyd; 1,1-Dichloracetaldehyd; Chloracetaldehyd; Acrolein; Crotonaldehyd; Propinal; Bromal; Valerianaldehyd; Cyclopropanal; Cyclopentanal; Cyclohexanal.

Bei der Durchführung des erfindungsgemässen Verfahrens werden ausserdem Halogenierungs- bzw. Sulfonylierungsmittel als Reaktionskomponenten benötigt. Als Halogenierungsmittel kommen vorzugsweise Phosphor- und Schwefelhalogenide, wie insbesondere -chloride und -bromide, in Frage. Hierzu gehören vorzugsweise Thionylchlorid, Sulfurylchlorid, Phosphortrichlorid, Phosphortribromid, Phosphoroxychlorid und Phosphorpentachlorid. Ausserdem sei vorzugsweise genannt: Phosgen. Als Sulfonylierungsmittel kommen vorzugsweise Alkyl- und Arylsulfohalogenide in Frage. Hierzu gehören vorzugsweise Methylsulfochlorid, Benzolsulfochlorid, p-Toluolsulfochlorid und p-Bromphenylsulfochlorid.

Als Lösungsmittel kommen für die erfindungsgemässe Umsetzung vorzugsweise organische Lösungsmittel in Frage, die gegen Pyrazol bzw. gegen die verwendeten Halogenierungs- und Sulfonylierungs-Mittel inert sind. Hierzu gehören vorzugsweise Ether, wie Diethylether oder Tetrahydrofuran; aromatische und aliphatische Kohlenwasserstoffe, wie Benzol, Toluol, Xylol oder Cyclohexan; halogenierte Kohlenwasserstoffe, wie Chloroform, Methylenchlorid, Tetrachlorkohlenstoff, 1,1,1-Trichlorethan, 1,2-Dichlorethan, 1,1,2-Trichlorethylen oder Chlorbenzol; sowie Formamide, wie insbesondere Dimethylformamid.

Die Reaktionstemperaturen können bei der Durchführung des erfindungsgemässen Verfahrens in einem grösseren Bereich variiert werden. Im allgemeinen arbeitet man zwischen -70 °C und +50 °C, vorzugsweise bei -20 °C bis +30 °C.

Bei der Durchführung des erfindungsgemässen Verfahrens setzt man auf 1 Mol Pyrazol der Formel (II) vorzugsweise 0,5 bis 2,0 Mol Aldehyd der Formel (III) ein und setzt das entstehende Hydroxy-pyrazol-Derivat der Formel (IV) direkt oder nach vorheriger Isolierung mit 0,5 bis 5 Mol Halogenierungs- bzw. Sulfonylierungs-Mittel um. Die Verbindungen der Formel (I) fallen dabei in Form ihrer Hydrohalogenide an und können so direkt nach üblichen Methoden isoliert werden. Es ist aber auch möglich, die Verbindungen der Formel (I) in üblicher Weise, wie z.B. durch Behandlung mit starken Basen, aus ihren Hydrohalogeniden in Freiheit zu setzen. Gegebenenfalls kann dann in bekannter Weise ein anderes Säureadditions-Salz hergestellt werden, z.B. durch Lösen einer Verbindung der Formel (I) in einem geeigneten inerten Lösungsmittel und Hinzufügen der Säure. Die Isolierung erfolgt im allgemeinen durch einfaches Abfiltrieren der Reaktionsprodukte.

In einer bevorzugten Ausführung des erfindungsgemässen Verfahrens kann man, ausgehend von erfindungsgemässen Verbindungen der Formel (I), in der Y für Chlor steht, durch allgemein bekannten Halogenaustausch (Finkelstein-Reaktion), wie z.B. durch Behandeln mit Kaliumbromid oder Kaliumjodid in Aceton, die entsprechenden Bromide und Jodide erhalten.

Zur Herstellung von Säureadditionssalzen der Verbindungen der Formel (I) kommen alle starken Säuren in Frage.

Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono-und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, sowie Sulfonsäuren, wie z.B. p-Toluolsulfonsäure und 1,5-Naphthalindisulfonsäure.

Die erfindungsgemässen substituierten Pyrazol-Derivate der Formel (I) eignen sich als Zwischenprodukte zur Synthese von substituierten N-Pyrazolylmethyl-halogenacetaniliden, welche herbizide Eigenschaften besitzen. Die substituierten N-Pyrazolylmethyl-halogenacetanilide der Formel

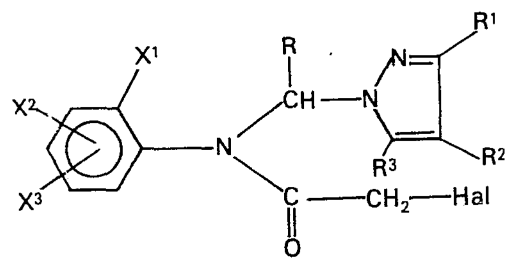

(V)

in welcher R, $R^1$, $R^2$ und $R^3$ die oben angegebene Bedeutung haben, Hal für Halogen steht und $X^1$, $X^2$ und $X^3$ gleich oder verschieden sind und für Wasserstoff oder Alkyl stehen, lassen sich herstellen, indem man Pyrazol-Derivate der Formel

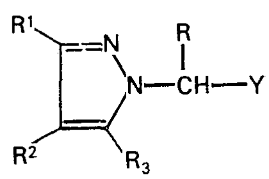

(I)

in welcher R, $R^1$, $R^2$, $R^3$ und Y die oben angegebene Bedeutung haben, mit Halogenacetaniliden der Formel

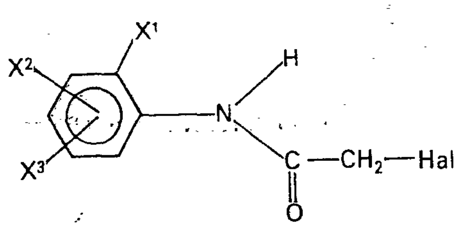

(VI)

in welcher Hal, $X^1$, $X^2$ und $X^3$ die oben angegebene Bedeutung haben, in Gegenwart eines Säurebindemittels und gegebenenfalls in Gegenwart eines organischen Lösungsmittels umsetzt, wobei die Pyrazol-Derivate der Formel (I) vorzugsweise in Form von Halogenwasserstoff-Salzen eingesetzt werden.

Die bei dieser Umsetzung als Ausgangsstoffe zu verwendenden Halogenacetanilide sind durch die Formel (VI) allgemein definiert. In dieser Formel steht Hal vorzugsweise für die Halogene Fluor, Chlor oder Brom, insbesondere Chlor oder Brom; $X^1$, $X^2$ und $X^3$ sind gleich oder verschieden und stehen vorzugsweise für Wasserstoff sowie geradkettiges oder verzweigtes Alkyl, wie insbesondere Methyl, Ethyl, Isopropyl, Isobutyl, sek.-Butyl oder tert.-Butyl.

Die Halogenacetanilide der Formel (VI) sind allgemein bekannte Verbindungen der organischen Chemie oder lassen sich nach bekannten Methoden herstellen. Als Beispiele seien genannt:

Chlor(brom)acetanilid; 2-Methyl-chlor(brom)acetanilid; 2-Ethyl-chlor(brom)acetanilid; 2-Isopropyl-chlor(brom)acetanilid; 2-sek.-Butyl-chlor(brom)acetanilid; 2-tert.-Butyl-chlor(brom)acetanilid; 2,6-Dimethyl-chlor(brom)acetanilid; 2,3-Dimethyl-chlor(brom)acetanilid; 2,5-Dimethyl-chlor(brom)acetanilid; 3,5-Dimethyl-chlor(brom)acetanilid; 2,6-Diethyl-chlor(brom)acetanilid; 2-Ethyl-6-methyl-chlor(brom)acetanilid; 2,3,4-Trimethyl-chlor(brom)acetanilid; 2,4,6-Trimethyl-chlor(brom)acetanilid; 2,4,5-Trimethyl-chlor(brom)acetanilid; 2,3,5-Trimethyl-chlor(brom)acetanilid; 2-Ethyl-4,6-dimethyl-chlor(brom)acetanilid; 2,6-Diethyl-4-methyl-chlor(brom)acetanilid; 2,6-Diisopropyl-4-methyl-chlor(brom)acetanilid.

Für die Umsetzung der erfindungsgemässen substituierten Pyrazol-Derivate der Formel (I) mit den Halogenacetaniliden der Formel (VI) kommen als Lösungsmittel alle inerten, mit Wasser nicht mischbaren, organischen Lösungsmittel in Frage. Hierzu gehören vorzugsweise Ether, wie Diethylether; aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das oben angegebene Verfahren zur Herstellung von substituierten N-Pyrazolylmethyl-halogenacetaniliden wird in Gegenwart von Säurebindemitteln durchgeführt. Als solche können alle üblichen Säurebindemittel verwendet werden. Hierzu gehören vorzugsweise anorganische Basen, wie beispielsweise Alkalihydroxide und Alkalicarbonate.

Die Reaktionstemperaturen können bei der Durchführung des oben angegebenen Verfahrens zur Herstellung von substituierten N-Pyrazolylmethyl-halogenacetaniliden in einem grösseren Bereich variiert werden. Im allgemeinen arbeitet man zwischen −70°C und +100°C, vorzugsweise zwischen −20°C und 80°C.

Bei der Durchführung des oben angegebenen Verfahrens setzt man vorzugsweise auf 1 Mol Halogenacetanilid der Formel (VI), 0,5 bis 2,0 Mol Pyrazolyl-Derivate der Formel (I) sowie 1 bis 10 Mol Säurebinder ein. Die Isolierung der Verbindungen der Formel (V) erfolgt in üblicher Weise.

In einer bevorzugten Ausführungsform wird das oben angegebene Verfahren zur Herstellung von substituierten N-Pyrazolylmethyl-halogenacetaniliden der Formel (V) in einem Zweiphasensystem, wie beispielsweise wässrige Natron- oder Kalilauge/Toluol oder Methylenchlorid, gegebenenfalls unter Zusatz von 0,01 bis 1 Mol eines Phasen-Transfer-Katalysators, wie beispielsweise Ammonium- oder Phosphoniumverbindungen, beispielsweise sei Benzyl-dodecyl-dimethyl-ammoniumchlorid (Zephirol) genannt, durchgeführt.

Die substituierten N-Pyrazolylmethyl-halogenacetanilide der Formel (V) zeichnen sich neben einer guten Totalherbizid-Wirkung vor allem durch ihre selektiven Einsatzmöglichkeiten in wichtigen Kulturpflanzen, wie Baumwolle, Rüben, Mais, Soja, Erdnüsse und Gemüse aus. Sie eignen sich vornehmlich zur pre-emergence-Anwendung, aber auch bei post-emergence-Anwendung zeigen sie Wirkung.

Die guten herbiziden Wirkungen der substituierten N-Pyrazolylmethyl-halogenacetanilide der Formel (V) gehen aus dem nachfolgenden Beispiel hervor.

Wirkstoffliste:

In dem nachfolgenden Beispiel werden die nachstehend angegebenen Wirkstoffe bezüglich ihrer herbiziden Wirkung getestet.

(A):

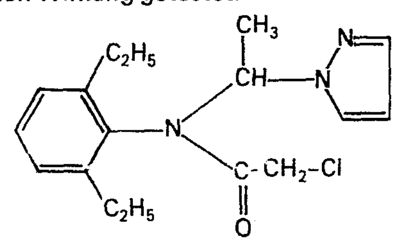

(B):

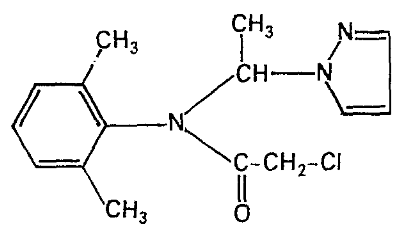

bekannt:

(C):

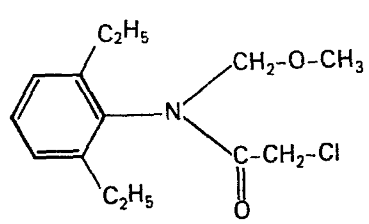

Beispiel A

Pre-emergence-Test

Lösungsmittel: 5 Gewichtsteile Aceton

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend dabei ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung.

Die substituierten N-Pyrazolylmethyl-halogenacetanilide der Formeln (A) und (B) zeigen in diesem Test eine bessere selektive herbizide Wirksamkeit als die aus dem Stand der Technik bekannte Substanz (C).

Herstellungsbeispiele

Beispiel 1

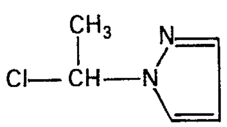

x HCl

Zu 340 g (5 Mol) Pyrazol in 1200 ml Methylenchlorid werden bei 0 bis 5 °C innerhalb einer Stunde 250 g (5,7 Mol) Acetaldehyd getropft. Man lässt ca. 1 Stunde bei 0 °C nachrühren. Das dabei entstehende N-(1-Hydroxyethyl)-pyrazol wird nicht isoliert, sondern die Reaktionslösung wird direkt bei 0 bis 5 °C innerhalb einer Stunde zu 1250 g (10,5 Mol) Thionylchlorid getropft. Man lässt 1 Stunde bei 20 °C nachrühren und engt dann bei 40 °C im Vakuum ein. Nach Zugabe von 300 ml Methylenchlorid wird erneut eingeengt. Der Rückstand wird im Vakuum destilliert. Man erhält 620,3 g (75% der Theorie) N-(1-Chlorethyl)-pyrazol-hydrochlorid vom Siedepunkt 55 °C /18 mm.

Umsetzung der Verbindung (1) zu dem entsprechenden substituierten N-Pyrazolylmethylhalogenacetanilid der Formel

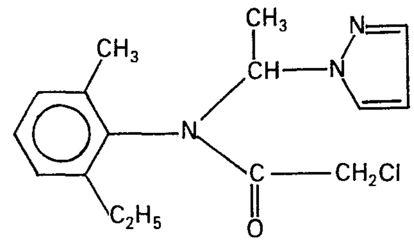

(V-1)

42,3 g (0,2 Mol) 2-Ethyl-6 methyl-chloracetanilid und 36,8 g (0,22 Mol) N-(1-Chlorethyl)-pyrazolhydrochlorid werden in 200 ml Methylenchlorid gelöst. Nach Zugabe von 0,5 ml Zephirol (50%ige wässrige Lösung von Benzyl-dodecyl-dimethylammoniumchlorid) tropft man unter starkem Rühren in eine Lösung von 80g (2 Mol) Natriumhydroxid in 80 ml Wasser ein, wobei sich das Reaktionsgemisch bis zum Rückfluss erhitzt. Man rührt ca. 3 Stunden weiter, bis das Reaktionsgemisch auf Raumtemperatur abgekühlt ist. Die organische Phase wird abgetrennt, mit Wasser neutral gewaschen, über Natriumsulfat getrocknet und im Vakuum eingeengt.

Man erhält ein farbloses Öl aus Rückstand, das nach einiger Zeit durchkristallisiert. Ausbeute 29g (47,5% der Theorie) 2-Ethyl-6-methyl-N-(pyrazol-1-yl-eth-1-yl)-chloracetanilid in Form weisser Kristalle vom Schmelzpunkt 74 °C. Die Substanz kann durch mehrfaches Umkristallisieren aus Diethylether gereinigt werden und hat dann einen Schmelzpunkt von 84 °C.

Herstellung des Vorproduktes:

(VI-1)

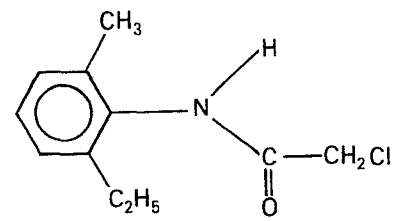

135,2 g (1 Mol) 2-Ethyl-6-methyl-anilin in 1000 ml Toluol werden mit 152 g (1,1 Mol) Kaliumcarbonat versetzt. Dazu werden unter Rühren 113 g (1 Mol) Chloressigsäurechlorid getropft. Nach Abklingen der exothermen Reaktion lässt man 2 Stunden unter Rückfluss nachrühren. Anschliessend wird das Reaktionsgemisch filtriert und das Filtrat im Vakuum auf 500 ml eingeengt. Die dabei entstehenden Kristalle werden abgesaugt und mit Petrolether gewaschen. Man erhält 202,9 g (96,2% der Theorie) 2-Ethyl-6-methylchloracetanilid in Form weisser Kristalle vom Schmelzpunkt 120 °C.

Beispiel 2

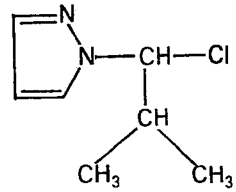

x HCl

An 204 g (3 Mol) Pyrazol gelöst in 400 ml Methylenchlorid werden bei 0-5 °C 220 g (3,2 Mol) Isobutylaldehyd zugetropft. Man lässt nach beendeter Zugabe noch zwei Stunden bei 0 °C nachrühren und tropft dann die Lösung bei 0-5 °C zu 750 g (6,3 Mol) Thionylchlorid. Man lässt die Reaktionsmischung anschliessend auf Raumtemperatur erwärmen und rührt noch 12 Stunden nach. Danach wird das Lösungsmittel im Vakuum abgezogen, der Rückstand in Tetrachlorkohlenstoff aufgenommen und kristallisiert. Die Kristalle werden abfiltriert, die Mutterlauge eingeengt und der Rückstand kristallisiert. Der so erhaltene Feststoff wird mit der ersten Fraktion vereinigt und mit 200 ml Tetrachlorkohlenstoff ausgerührt. Danach filtriert man ab, wäscht mit 100 ml Tetrachlorkohlenstoff nach und trocknet den Feststoff bei 40 °C im Vakuum. Man erhält 530 g 1-(1'-Chlor-isobutyl)-pyrazol-hydrochlorid (91% der Theorie) vom Schmelzpunkt 110-114 °C.

## Beispiel 3

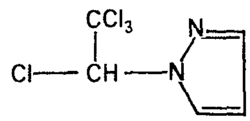 x HCl

Zu 68 g (1 Mol) Pyrazol in 400 ml Methylenchlorid werden bei 0 bis 5 °C innerhalb einer Stunde 175 g (1,2 Mol) Chloral zugetropft. Man lässt 3 Stunden bei 25 °C nachrühren und saugt das Kristallisat ab. Die Mutterlauge wird eingeengt und weiteres Kristallisat erneut abgesaugt. Die vereinigten Kristallisate werden in Methylenchlorid gelöst und bei 10 °C innerhalb einer halben Stunde zu 250 g (2,1 Mol) Thionylchlorid in 500 ml Methylenchlorid gegeben. Man lässt 18 Stunden bei Raumtemperatur nachrühren. Anschliessend wird das Reaktionsgemisch bei 50 °C im Vakuum eingeengt. Der Rückstand wird mit 200 ml Chloroform versetzt und erneut eingeengt. Nach Zugabe von 200 ml Tetrachlorkohlenstoff kristallisiert das Produkt aus; es wird abgesaugt und getrocknet. Man erhält 178 g (66% der Theorie) N-(1,2,2,2-Tetrachlorethyl)-pyrazol-hydrochlorid vom Schmelzpunkt 95 °C.

In entsprechender Weise werden diejenigen Verbindungen erhalten, die in der Tabelle 1 formelmässig aufgeführt sind.

Tabelle 1

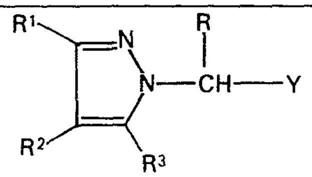 (I)

| Bsp.Nr. | R | R¹ | R² | R³ | Y | Schmelzpunkt (°C) Siedepunkt (°C) |
|---|---|---|---|---|---|---|
| 4 | $-CH_3$ | $CH_3$ | Cl | $CH_3$ | Cl | Öl (xHCl) |
| 5 | $-CH_3$ | H | Cl | H | Cl | Sdp: 60-62/$_{20mb}$ (xHCl) |
| 6 | $-C_2H_5$ | H | H | H | Cl | Öl (xHCl) |
| 7 | $-n-C_3H_7$ | H | H | H | Cl | Öl (xHCl) |
| 8 | $-CH_3$ | H | H | H | Br | Sdp: 60-65/$_{20mb}$ |
| 9 | $-CH_3$ | H | H | H | Cl | Sdp: 50-55/$_{20mb}$ |

Nach dem in der Anmeldung beschriebenen Verfahren werden die in der nachstehenden Tabelle 2 formelmässig aufgeführten Zwischenprodukte der Formel (IV) erhalten.

Tabelle 2

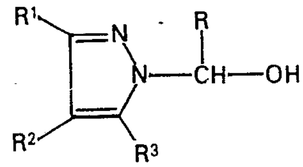

(IV)

| Bsp.Nr. | R | $R^1$ | $R^2$ | $R^3$ | Physikalische Konstante |
|---|---|---|---|---|---|
| IV-1 | $CH_3$ | H | H | H | nicht isoliert |
| IV-2 | i-$C_3H_7$ | H | H | H | nicht isoliert |
| IV-3 | $-CCl_3$ | H | H | H | Fp: 109 °C |
| IV-4 | $CH_3$ | $CH_3$ | Cl | $CH_3$ | Fp: 39–44 °C |

Aus den erfindungsgemässen Stoffen der Formel (I) werden gemäss Beispiel (1) durch Umsetzung mit Halogenacetaniliden der Formel (VI) die in der nachstehenden Tabelle 3 formelmässig aufgeführten Verbindungen erhalten.

Tabelle 3

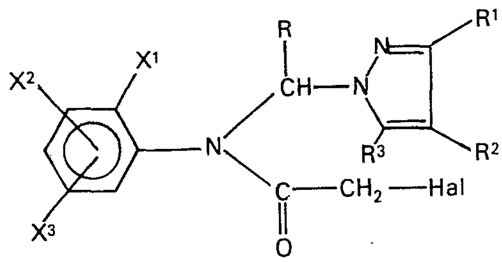

(V)

| Bsp.Nr. | $X^1$ | $X^2$ | $X^3$ | R | $R^1$ | $R^2$ | $R^3$ | Hal | Schmelzpunkt (°C) |
|---|---|---|---|---|---|---|---|---|---|
| V-2 | $CH_3$ | 6-$CH_3$ | H | $CH_3$ | H | H | H | Cl | 90 |
| V-3 | $C_2H_5$ | 6-$C_2H_5$ | H | $CH_3$ | H | H | H | Cl | 80 |
| V-4 | $CH_3$ | 6-$CH_3$ | H | $CH_3$ | H | H | H | Cl | 95 (xHCl) |

Die benötigten Vorprodukte der Formel (VI) werden nach der in Beispiel 1 angegebenen Methode hergestellt:

Tabelle 4

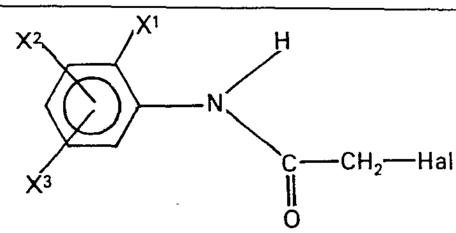

(VI)

| Bsp.Nr. | $X^1$ | $X^2$ | $X^3$ | Hal | Schmelzpunkt (°C) |
|---|---|---|---|---|---|
| VI- 2 | $CH_3$ | 6-$CH_3$ | H | Cl | 148 |
| VI- 3 | $C_2H_5$ | 6-$C_2H_5$ | H | Cl | 133 |
| VI- 4 | i-$C_3H_7$ | H | H | Cl | 79 |
| VI- 5 | tert.-$C_4H_9$ | H | H | Cl | 96 |
| VI- 6 | $C_2H_5$ | H | H | Cl | 103 |
| VI- 7 | $CH_3$ | H | H | Cl | 109 |
| VI- 8 | $CH_3$ | 3-$CH_3$ | H | Cl | 135 |
| VI- 9 | $CH_3$ | 5-$CH_3$ | H | Cl | 154 |
| VI-10 | $CH_3$ | 4-$CH_3$ | 6-$CH_3$ | Cl | 177 |
| VI-11 | $C_2H_5$ | 4-$CH_3$ | 6-$CH_3$ | Cl | 134 |
| VI-12 | sek.-$C_4H_9$ | H | H | Cl | Öl |
| VI-13 | H | H | H | Cl | 132 |

**Patentansprüche**

1. Substituierte Pyrazol-Derivate der Formel

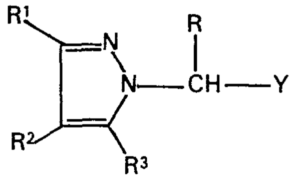

(I)

in welcher R für Alkyl mit 1 bis 8 Kohlenstoffatomen, Cycloalkyl mit 3 bis 6 Kohlenstoffatomen, Halogenalkyl mit bis zu 2 Kohlenstoffatomen und bis zu 3 Halogenatomen, Alkoxyalkyl mit 1 bis 4 Kohlenstoffatomen im Alkylteil und 1 bis 4 Kohlenstoffatomen im Alkoxyteil, für Alkenyl mit 2 bis 4 Kohlenstoffatomen, Alkinyl mit 2 bis 4 Kohlenstoffatomen oder für gegebenenfalls durch Halogen, Alkyl mit 1 bis 4 Kohlenstoffatomen, Halogenalkyl mit bis zu 2 Kohlenstoffatomen und bis zu 3 Halogenatomen, Alkoxy mit bis zu 2 Kohlenstoffatomen, Alkylthio mit bis zu 2 Kohlenstoffatomen, Cyano und/oder Nitro substituiertes Phenyl steht, $R^1$, $R^2$ und $R^3$ gleich oder verschieden sind und für Wasserstoff, Alkyl mit 1 bis 4 Kohlenstoffatomen, Halogen oder Alkoxy mit 1 bis 4 Kohlenstoffatomen stehen und Y für Fluor, Chlor, Brom, Jod, Alkylsulfonyl mit 1 bis 4 Kohlenstoffatomen im Alkylteil oder für gegebenenfalls durch Alkyl mit 1 bis 4 Kohlenstoffatomen und/oder Halogen substituiertes Phenylsulfonyl steht, und deren Säureadditionssalze.

2. Verfahren zur Herstellung von substituierten Pyrazol-Derivaten der Formel (I) gemäss Anspruch 1, dadurch gekennzeichnet, dass man Pyrazole der Formel

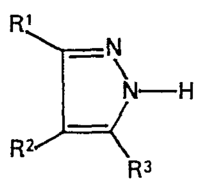

(II)

in welcher $R^1$, $R^2$ und $R^3$ die oben angegebene Bedeutung haben, mit Aldehyden der Formel

$$O = CH - R \qquad (III)$$

in welcher R die oben angegebene Bedeutung hat, gegebenenfalls in Gegenwart eines Lösungsmittels umsetzt und die dabei entstehenden Hydroxy-pyrazol-Derivate der Formel

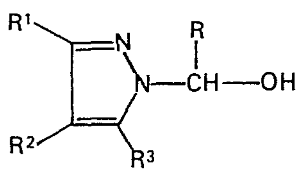

(IV)

in welcher R, $R^1$, $R^2$ und $R^3$ die oben angegebene Bedeutung haben, direkt oder gegebenenfalls nach Isolierung mit einem Halogenierungsmittel bzw. einem Sulfonylierungsmittel, gegebenenfalls in Gegenwart eines Lösungsmittels umsetzt.

3. Verbindungen der Formel

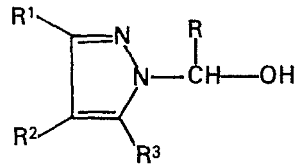

(IVa)

in welcher R für Alkyl mit 1 bis 8 Kohlenstoffatomen oder für Halogenalkyl mit bis zu 2 Kohlenstoffatomen und bis zu 3 Halogenatomen steht, $R^1$ für Wasserstoff oder Alkyl mit 1 bis 4 Kohlenstoffatomen steht, $R^2$ für Wasserstoff oder Halogen steht und $R^3$ für Wasserstoff oder Alkyl mit 1 bis 4 Kohlenstoffatomen steht.

**Revendications**

1. Dérivés substitués du pyrazole, de formule:

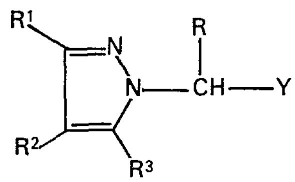

(I)

dans laquelle: R représente un groupe alkyle en $C_1$-$C_8$, cycloalkyle en $C_3$-$C_6$, halogénoalkyle contenant jusqu'à 2 atomes de carbone et jusqu'à 3 atomes d'halogènes, alcoxy-alkyle contenant 1 à 4 atomes de carbone dans la partie alkyle et 1 à 4 atomes de carbone dans la partie alcoxy, alcényle en $C_2$-$C_4$, alcynyle en $C_2$-$C_4$ ou phényle éventuellement substitué par des halogènes, des groupes alkyle en $C_1$-$C_4$, halogénoalkyle contenant jusqu'à 2 atomes de carbone et jusqu'à 3 atomes d'halogènes, alcoxy contenant jusqu'à 2 atomes de carbone, alkylthio contenant jusqu'à 2 atomes de carbone, cyano et/ou nitro, $R^1$, $R^2$ et $R^3$, identiques ou différents, représentent l'hydrogène, des groupes alkyle en $C_1$-$C_4$, des halogènes ou des groupes alcoxy en $C_1$-$C_4$ et Y représente le fluor, le chlore, le brome, l'iode, un groupe alkylsulfonyle contenant de 1 à 4 atomes de carbone dans la partie alkyle ou un groupe phénylsulfonyle éventuellement substitué par des groupes alkyle en $C_1$-$C_4$ et/ou des halogènes, et leurs sels formés par addition avec des acides.

2. Procédé de préparation des dérivés substitués du pyrazole de formule I selon la revendication 1, caractérisé en ce que l'on fait réagir des pyrazoles de formule:

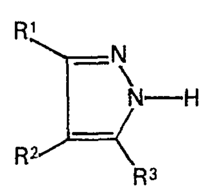

(II)

dans laquelle $R^1$, $R^2$ et $R^3$ ont les significations indiquées ci-dessus, avec des aldéhydes de formule:

$$O = CH - R \qquad (III)$$

dans laquelle R a les significations indiquées ci-dessus, le cas échéant en présence d'un solvant, et on fait réagir les dérivés d'hydroxypyrazole ainsi obtenus, de formule:

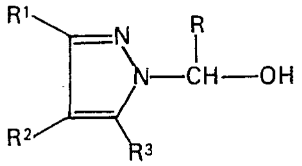

(IV)

dans laquelle R, $R^1$, $R^2$ et $R^3$ ont les significations indiquées ci-dessus, directement ou, le cas échéant, après les avoir isolés, avec un agent halogénant ou un agent sulfonylant respectivement, éventuellement en présence d'un solvant.

3. Composés de formule:

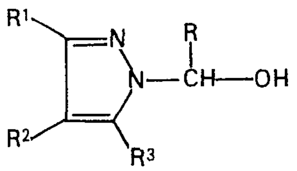

(IVa)

dans laquelle: R représente un groupe alkyle en $C_1$-$C_8$ ou halogénoalkyle contenant jusqu'à 2 atomes de carbone et jusqu'à 3 atomes d'halogènes, $R^1$ représente l'hydrogène ou un groupe alkyle en $C_1$-$C_4$, $R^2$ représente l'hydrogène ou un halogène et $R^3$ représente l'hydrogène ou un groupe alkyle en $C_1$-$C_4$.

**Claims**

1. Substituted pyrazole derivatives of the formula

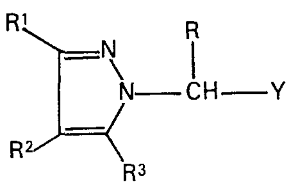

(I)

in which R represents alkyl with 1 to 8 carbon atoms, cycloalkyl with 3 to 6 carbon atoms, halogenoalkyl with up to 2 carbon atoms and up to 3 halogen atoms, alkoxyalkyl with 1 to 4 carbon atoms in the alkyl part and 1 to 4 carbon atoms in the alkoxy part, alkenyl with 2 to 4 carbon atoms, alkynyl with 2 to 4 carbon atoms or phenyl optionally substituted by halogen, alkyl with 1 to 4 carbon atoms, halogenoalkyl with up to 2 carbon atoms and up to 3 halogen atoms, alkoxy with up to 2 carbon atoms, alkylthio with up to 2 carbon atoms, cyano and/or nitro, $R^1$, $R^2$ and $R^3$ are identical or different and represent hydrogen, alkyl with 1 to 4 carbon atoms, halogen or alkoxy with 1 to 4 carbon atoms and Y represents fluorine, chlorine, bromine, iodine, alkylsulphonyl with 1 to 4 carbon atoms in the alkyl part or phenylsulphonyl optionally substituted by alkyl with 1 to 4 carbon atoms and/or halogen, and acid addition salts thereof.

2. Process for the preparation of substituted pyrazole derivatives of the formula (I) according to Claim 1, characterised in that pyrazoles of the formula

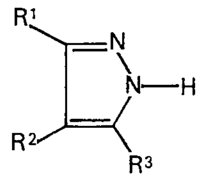

(II)

in which $R^1$, $R^2$ and $R^3$ have the meaning given above, are reacted with aldehydes of the formula

$$O = CH - R \qquad (III)$$

in which R has the meaning given above, if appropriate in the presence of a solvent and the hydroxypyrazole derivatives thereby formed of the formula

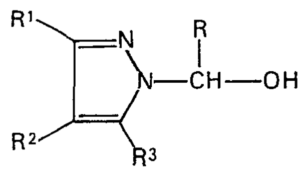

(IV)

in which R, $R^1$, $R^2$ and $R^3$ have the meaning given above, are reacted directly or if appropriate after being isolated with a halogenating agent or a sulphonylating agent, if appropriate in the presence of a solvent.

3. Compounds of the formula

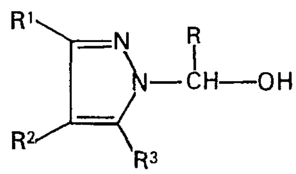

(IVa)

in which R represents alkyl with 1 to 8 carbon atoms or halogenoalkyl with up to 2 carbon atoms and up to 3 halogen atoms, $R^1$ represents hydrogen or alkyl with 1 to 4 carbon atoms, $R^2$ represents hydrogen or halogen and $R^3$ represents hydrogen or alkyl with 1 to 4 carbon atoms.